# EUROPEAN PATENT APPLICATION

(11) **EP 1 707 502 A1**
(43) Date of publication of application: **04.10.2006**
(21) Application number: 06075727.5
(22) Date of filing: 29.03.2006
(51) Int. Cl.: B65D 83/16, B65D 83/14

(54) **Device for spraying a liquid**

(30) Priority: 29.03.2005 BE 200500164
(71) Applicant: Nijs, Frank Alexander Maurice, 2110 Wijnegem (BE)
(72) Inventor: Nijs, Frank Alexander Maurice, 2110 Wijnegem (BE)
(74) Representative: Donné, Eddy

(57) **Abstract**

Device for spraying a liquid, characterised in that it mainly consists of a holder (8) in which can be provided one or several liquid containers (9) under pressure with a sprinkler head (12), whereby this holder (8) can be moved in a guide (5) and is provided with means to activate the sprinkler head (12) of one or several of the above-mentioned liquid containers (9).

## Description

The present invention concerns a device for spraying a liquid, in particular what is called a "sunless tanning" lotion on a person's body.

Devices for applying tanning lotions on the human body are already known.

Such known devices mainly consist of a shower cubicle in which are provided one or several sprayers connected to a reservoir for the above-mentioned lotion.

The known devices further comprise a compressor which makes it possible to generate a required pressure in the above-mentioned reservoir so as to be able to spray the lotion in the appropriate amounts.

The above-mentioned sprayers can be provided in the shower cubicle either or not in a movable manner, whereby the sprayers make an up-and-down movement in the shower cubicle while spraying the lotion so as to be able to evenly apply the lotion over the entire body of the person in the shower cubicle.

A disadvantage of the above-described devices is that it is impossible to regularly spray another liquid through the sprayers.

Indeed, each time another liquid needs to be sprayed, the sprayers and the pipes connecting these sprayers to the reservoir need to be washed.

Moreover, the above-mentioned sprayers need to be cleaned regularly so as to avoid obstructions and/or possible fouling thereof.

Another disadvantage is that the above-mentioned reservoirs are usually difficult to handle due to their design and weight, and that moreover changing the reservoirs is relatively time-consuming and can only be done by someone who has the required tools.

Moreover, the pressure on the reservoirs needs to be regularly checked and adjusted if necessary, so as to obtain a good spraying of the lotion.

Another disadvantage of the above-described devices is that they are relatively expensive.

The present invention aims to remedy the above-mentioned and other disadvantages.

To this end, the invention concerns a device for spraying a liquid which mainly consists of a holder in which can be provided one or several liquid containers under pressure with a sprinkler head, whereby this holder can be moved in a guide and is provided with means to activate the sprinkler head of one or several of the above-mentioned liquid containers.

An advantage of the device according to the invention is that it allows to successively spray different liquids without any risk of traces of previously sprayed liquids remaining in the last sprayed liquid.

Another advantage of the present invention is that it is extremely user-friendly.

Indeed, as the liquid reservoirs in this case consist of containers in the form of spray cans that are easy to handle, they can be easily and quickly changed or replaced by the user himself, without having to extensively clean any possible pipes or the like and without any tools being required.

Another advantage is that the sprinkler heads are part of the exchangeable liquid containers, such that these sprinkler heads do not need to be maintained, which is also advantageous from a hygienic point of view.

Another advantage is that the device according to the invention can be made relatively cheap and compact, compared to the existing devices.

In order to better explain the characteristics of the invention, the following preferred embodiment of a device according to the invention for spraying a liquid is given as an example only without being limitative in any way, with reference to the accompanying drawings, in which:
figure 1 represents a cubicle in which is provided a device according to the invention;
figure 2 represents a device according to the invention to a larger scale;
figure 3 represents a section according to line III-III in figure 1 to a larger scale;
figure 4 represents the part indicated by F4 in figure 3 to a larger scale;
figure 5 represents a variant of figure 2;
figure 6 represents a section according to line VI-VI in figure 5.

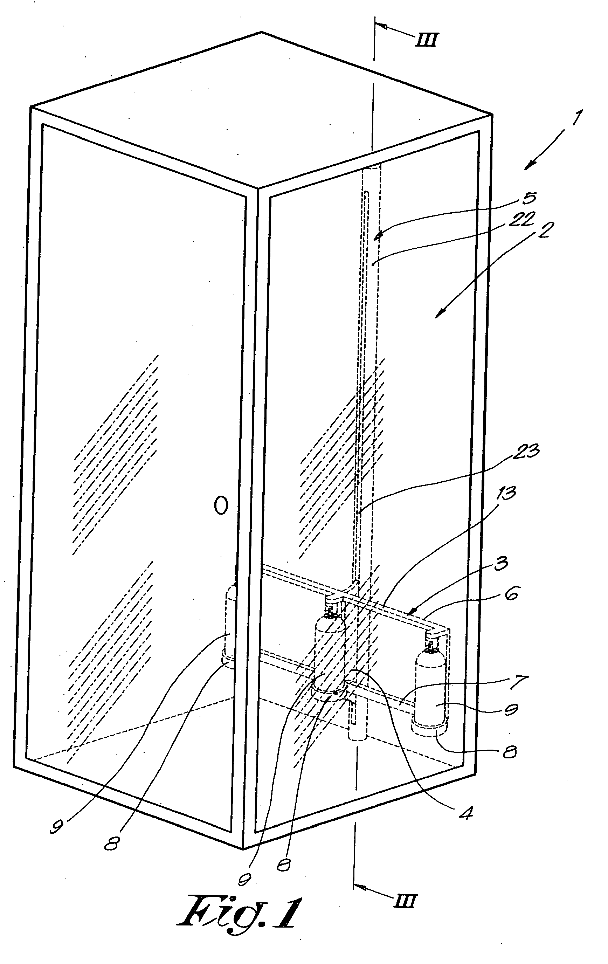

Figure 1 represents a cubicle 1 in which is provided a device 2 according to the invention for spraying a liquid.

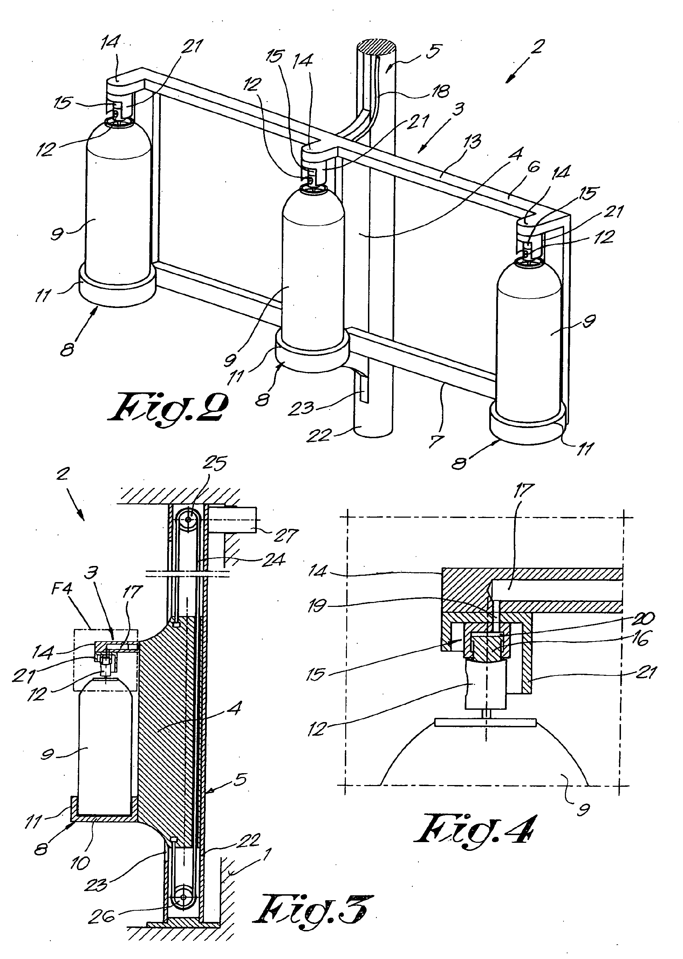

As is represented in figure 2, this device 2 mainly consists of a frame 3 which is fixed to a carriage 4 provided in a movable manner in a guide 5.

The above-mentioned framework 3 mainly consists of a frame 6 on which are provided, on a lower edge 7, in this case three holders 8 for liquid containers 9.

These holders 8 in this case each consist of a plate 10 which is provided crosswise on the frame 6 and which is provided with a standing edge 11 on its peripheral edge.

The device 2 according to the invention further comprises means to activate a sprinkler head 12 of each of the above-mentioned liquid containers 9.

In the embodiment of the invention represented in the figures, these means consist of a press-on mechanism which can be moved to and fro between a position in which the press-on mechanism pushes in the sprinkler head 12 of the liquid container 9 concerned and a position in which the press-on mechanism of the sprinkler head 12 has been removed.

The above-mentioned means are in this case provided on the top edge 13, opposite the above-mentioned edge 7 on which the holders 8 are provided.

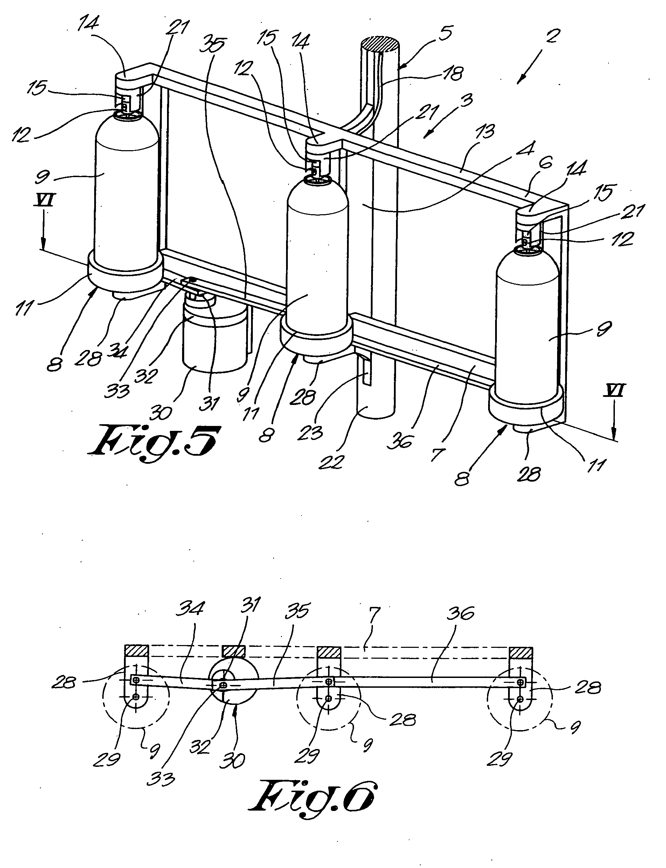

As is represented in figure 4, the above-mentioned means in this case consist of a protrusion 14 provided crosswise on the frame 6, at the free end of which is provided a bush 15 in which is provided a pen 16 as an axially moveable piston.

In the above-mentioned protrusion 14 is provided a bore hole 17 which is connected to a compressed air line 18 on the one hand and which is connected to a chamber 20 on the other hand via a channel 19 which is confined by the bush 15 and the pen 16.

A protective cap 21 as represented in the figures can be possibly provided between the above-mentioned protrusion 14 and the above-mentioned bush 15, whereby the channel 19 extends through this protective cap 21.

As is represented in figure 3, the above-mentioned guide 5 consists of a strut 22, a standing strut in this case, in which is provided a groove 23 in which the above-mentioned carriage 4 is provided in a moving manner.

The device 2 according to the invention is provided with means to move the above-mentioned carriage 4 in the guide 5, which means in this case consist of a cable 24 which is anchored in the carriage 4 with both far ends and which is guided over rollers 25,26 on both opposite far ends of the guide 5.

It is clear that at least one of the above-mentioned rollers 25-26 is connected to the outgoing shaft of a drive 27.

Finally, the liquid containers 9 are in this case spray cans which, as is known, are provided with a sprinkler head 12 which can be activated by simply pressing on it.

The working of the above-described device 2 according to the invention is simple and as follows.

In a starting position, the carriage 4 is preferably situated on one far end of the guide 5, and a filled liquid container 9 is provided in one or several of the above-mentioned holders 8, such that these liquid containers 9 are positioned opposite an above-mentioned pen 16 with their sprinkler head 12.

As soon as the device 2 is activated, the above-mentioned drive 27 of one of the rollers 25-26 is started, whereupon the carriage 4 moves in the guide 5.

In the meantime, compressed air is supplied via the compressed air line 18 into the chamber 20 of the bush 15, as a result of which the pen 16 is extended and thereby exerts a pressure on the sprinkler head 12 of the liquid container 9 concerned, as a result of which the liquid in this container 3 is sprayed through the sprinkler head 12.

Once the carriage 4 has reached a desired position, the run of the rollers 25-26 can be turned round, such that also the direction of movement of the carriage 4 is turned round and the carriage 4 is moved into its original position again, while the pressure in the compressed air line 18 can be either or not maintained.

For a subsequent work cycle, the above-mentioned liquid containers 9 can be either or not replaced or filled up.

It is clear that the movement of the carriage 4 can be easily set during a single work cycle by means of a control for the above-mentioned drive.

It should be noted that the above-mentioned framework 3 can be made according to all sorts of variants, for example in the shape of a horizontally positioned whole or half ring, whereby the sprinkler heads 12 of the different liquid containers 9 provided therein are directed towards the centre of the ring, so as to cover a person entirely with the appropriate liquid in a single movement of the carriage 4, without this person having to turn round.

Also the guide 5 can be made according to different variants, such as for example a variant in which a carriage 4 is provided which can be moved up and down by means of compressed air.

Of course, it is also possible to provide liquid containers 9 in the different holders 8 in which are provided different liquids.

Further, it should be noted that, in contrast with the embodiment represented in the figures, the guide 5 must not be provided centrally behind the framework 3, but it may also be situated next to the framework 3, whereby the carriage 4 in this case can be fixed to an edge of the framework.

Moreover, the device according to the invention can be fixed to all sorts of walls, either or not in a cubicle 1. An additional advantage thereof is that the device according to the invention can also be provided on a mobile wall, as a result of which the device according to the invention can be simply and quickly moved.

Finally, it should be noted that the means for activating the sprinkler head 12 of one or several of the above-mentioned liquid containers 9 may also be mechanical and may for example consist of a lath to be manually operated, on which are provided pens 16 which are positioned opposite the above-mentioned sprinkler heads 12.

Figures 5 and 6 represent a variant of a device according to the invention for spraying a liquid, which is equipped with means to make the liquid containers 9 rotate.

These means in this case consist of three crosswise protrusions 23 which are provided on the lower edge 7 of the above-mentioned frame 6, whereby the above-mentioned holders 8 can rotate on said crosswise protrusions 28 by means of a shaft 29.

Further, a motor 30 is fixed to the frame 6 whose outgoing shaft 31 drives a disc 32 on which is eccentrically provided a pen 33.

To the above-mentioned pen 33 are in this case fixed two rods 34,35 which are fixed to the above-mentioned holders 8 with their free ends, at a mutual distance from the above-mentioned shaft 29.

A third rod 36 connects the above-mentioned pens 33 of one of the above-mentioned holders 8 to the third holder 8 in an analogous manner.

The working of this variant differs from the working of the device represented in figures 1 to 4 in that, when the disc 32 is driven by the motor 30, the holders 8 are turned to and fro round the shaft 29 concerned with which they are fixed to the crosswise protrusions 28 of the frame 6.

As the holders 8 are turned, also the liquid containers 9 are turned to and fro, as a result of which the sprayed liquid can be evenly distributed in a horizontal plane.

This even distribution of the sprayed liquid offers the additional advantage that an even layer of lotion will be sprayed on a person taking place in the cubicle 1, such that the sunless tanning can provide an optimal result.

The present invention is by no means limited to the above-described embodiment represented in the accompanying drawings; on the contrary, such a device according to the invention for spraying a liquid can be made according to many variants while still remaining within the scope of the invention.

## Claims

1. Device for spraying a liquid, **characterised in that** it mainly consists of a holder (8) in which can be provided one or several liquid containers (9) under pressure with a sprinkler head (12), whereby this holder (8) can be moved in a guide (5) and is provided with means to activate the sprinkler head (12) of one or several of the above-mentioned liquid containers (9).

2. Device according to claim 1, **characterised in that** the above-mentioned means consist of a press-on mechanism which can be moved to and fro between a position in which the press-on mechanism pushes in the sprinkler head (12) of the liquid container (9) concerned and a position in which the press-on mechanism has been removed from the sprinkler head (12).

3. Device according to claim 2, **characterised in that** the press-on mechanism is driven by means of compressed air.

4. Device according to claim 2, **characterised in that** the press-on mechanism contains an element (14) on which is provided a cylinder (15) in which is provided a piston (16) in a movable manner.

5. Device according to claim 4, **characterised in that** in the above-mentioned element (14) is provided a bore hole (17) which is connected to a compressed air line (18) on the one hand and to a channel (19) on the other hand which opens in a chamber (20) in the cylinder (15), behind the piston (16).

6. Device according to any one of the preceding claims, **characterised in that** the holders (8) are designed to provide spray cans in them.

7. Device according to claim 1, **characterised in that** it contains means to make the liquid containers (9) rotate.

8. Device according to claim 1, **characterised in that** the above-mentioned holders (3) can rotate in relation to the guide (5) by means of a shaft (29).

9. Device according to claim 8, **characterised in that** the above-mentioned means for making the liquid containers (9) rotate consist of at least one rod (34,35) which is eccentrically connected to a disc (32) with one far end, and which is connected to one of the above-mentioned holders (8) with its other far end at a mutual distance from the above-mentioned shaft (29).
